Europäisches Patentamt
European Patent Office
Office européen des brevets

(19)

(11) Publication number: **0 048 301**
**A1**

(12) # EUROPEAN PATENT APPLICATION

(21) Application number: 80105739.9

(22) Date of filing: 24.09.80

(51) Int. Cl.[3]: C 07 C 149/243
C 07 F 9/38, A 61 K 31/195
A 61 K 31/66

(43) Date of publication of application:
31.03.82 Bulletin 82/13

(84) Designated Contracting States:
AT BE CH DE FR GB IT LI LU NL SE

(71) Applicant: MERCK & CO. INC.
126, East Lincoln Avenue P.O. Box 2000
Rahway, New Jersey 07065(US)

(72) Inventor: Graham, Donald W.
294 Old Tote Road
Mountainside New Jersey 07092(US)

(72) Inventor: Rogers, Edward F.
61 Kings Highway
Middletown New Jersey 07748(US)

(72) Inventor: Kahan, Frederick M.
2022 Brookside Avenue
Scotch Plains New Jersey 07076(US)

(74) Representative: Abitz, Walter, Dr.-Ing. et al,
Abitz, Morf, Gritschneder P.O. Box 86 01 09
D-8000 München 86(DE)

(54) **2-(Cyclopropane-carboxamido)-2-alkenoic acids, their esters and salts, and antibacterial compositions comprising the same and a thienamycin-type compound.**

(57) ω-(2-Amino-2-carboxyethylthio)-2-(cyclopropane-carboxamido)-2-alkenoic acids, their esters and salts of the formula

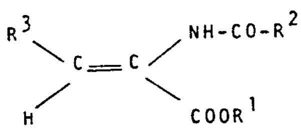

in the Z configuration, wherein $R^2$ is 2,2-dimethylcyclopropyl or 2,2-dichlorocyclopropyl; $R^1$ is hydrogen, loweralkyl of 1-6 carbon atoms, dialkylaminoalkyl with 1-6 carbon atoms in each alkyl group, or a pharmaceutically acceptable cation; $R^3$ is an alkylene chain of 3-7 carbon atoms, having a terminal substituent which is 2-amino-2-carboxyethylthio, or 1-(phosphono)ethylamino which selectively inhibit the metabolism of dipeptidase (E.C.3.4.13.11) and therefore are useful in combination with antibacterial products, by preference with the thienamycin class of compounds.

EP 0 048 301 A1

TITLE MODIFIED
see front page

16140YA

Z-ω-(2-AMINO-2-CARBOXYETHYLTHIO)-2-(CYCLOPROPANE-CARBOXAMIDO)-2-ALKENOIC ACIDS, THEIR ESTERS AND SALTS AND ANTIBACTERIAL COMPOSITIONS COMPRISING THE SAME AND A THIENAMYCIN TYPE COMPOUND

INTRODUCTION:

A new class of fused ring β-lactam antibiotics, including thienamycin and its semisynthetic derivatives, epithienamycins, and olivanic acids, has recently been described. These compounds which will be defined more extensively below, are hereinafter referred to as the "thienamycin class of compounds". These compounds have a high level of antibacterial activity, but are subject to extensive metabolism by mammalian species.

The kidney was identified as the primary site of metabolism, and an enzyme was purified from renal extracts which catalyzed the inactivation of thienamycin by hydrolysis of the β-lactam. By such criteria as cytological localization, substrate specificity and susceptibility to enzyme inhibitors, this enzyme is very similar if not identical to a widely studied renal dipeptidase (E.C.3.4.13.11), also referred to in the literature as "dehydropeptidase-I". However, the β-lactamase activity is exhibited only toward the thienamycin class of compounds. Indeed, there exists no precedent example of the mammalian metabolism via β-lactam cleavage of any representative of the classical β-lactam antibiotics, the penicillins and cephalosporins.

DESCRIPTION OF THE INVENTION:

The chemical substance which selectively inhibits the metabolism of the dipeptidase /E.C.3.4.13.11/, also called "dipeptidase inhibitors", are compounds of the general formula

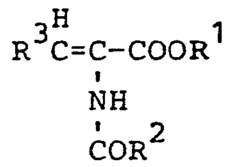

I

in the Z configuration wherein $R^2$ is 2,2-dimethylcyclopropyl; $R^1$ is hydrogen, loweralkyl of 1-6 carbon atoms, dialkylaminoalkyl with 1-6 carbon atoms in each alkyl group, or a pharmaceutically acceptable cation; $R^3$ is an alkylene chain of 3-7 carbon atoms, having a terminal substituent which is 2-amino-2-carboxyethylthio or 1-(phosphono)ethylamino.

Especially preferred are Z-8-(L-2-amino-2-carboxyethylthio)-2-(2,2-dimethylcyclopropanecarboxamido)-2-octenoic acid and Z-7-(L-2-amino-2-carboxyethylthio)-2-(2,2-dimethylcyclopropanecarboxamido)-2-heptenoic acid, their lower alkyl and dialkylaminoalkylesters and their salts with pharmaceutically acceptable bases.

The Z configuration (J.E. Blackwood et al., J. Am. Chem. Soc., 90, p. 509 (1968)) is assigned to the above compounds on the basis of their NMR spectra by analogy with the work of A. Srinavasan et al. /Tetrahedron Lett., 891 (1976)/.

Various pharmaceutically acceptable derivatives such as alkali and alkaline earth metal, ammonium, or amine salts, can be employed. Salts such as the sodium, potassium, calcium, magnesium, or tetramethylammonium salts are suitable.

UTILITY OF THE INVENTION:

As noted above, the compounds of this invention are dipeptidase (E.C.3.4.13.11) inhibitors, and can be used in combination with antibacterial compounds which are subject to renal degradation. The group of antibiotics of present primary importance for use in combination with the compounds of this invention are the "thienamycin class of compounds".

The term "thienamycin class of compounds" is used to identify any of a number of naturally occurring, semi-synthetic, or synthetic derivatives or analog compounds having a common fused-ring β-lactam nucleus. These compounds can be generically classed as 6- and (optionally) 2-substituted pen-2-em-3-carboxylic acids and 1-carbadethia-pen-2-em-3-carboxylic acids or 1-azabicyclo[3.2.0]hept-2-ene-7-one-2-carboxylic acids.

Specific compounds particularly useful in this invention are represented structurally in the following formula II:

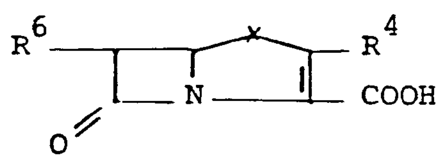

II

wherein X can be $CH_2$ or S; $R^4$ can be hydrogen; $-S-CH_2CH_2NHR^5$, wherein $R^5$ is hydrogen, acetyl, formimidoyl, acetimidoyl; $-S(O)-CH=CHNHCOCH_3$ and $-S-CH=CHNHCOCH_3$; and $R^6$ is $-\underset{\mid\,R^{7}}{CH}CH_3$ wherein $R^7$ is hydrogen, hydroxy or sulfonyloxy, or $R^6$ is H. All possible stereoisomeric forms are included within the above structural definition.

All of these compounds within Formula II are described in the literature. When X is $CH_2$, and $R^4$ is $SCH_2CH_2NH_2$, and $R^6$ is $CH(OH)CH_3$, the compound is known as thienamycin, an antibiotic produced by fermentation of S. cattleya, deposited as NRRL 8057 at Northern Regional Research Laboratories, U.S. Department of Agriculture, Peoria, Illinois, U.S.A., on November 18, 1974 described in U.S. Patent 3,950,357, issued April 13, 1976. The fermentation product N-acetyl thienamycin ($R^6$ is $CH(OH)CH_3$, and $R^5$ is acetyl), also called 924A, is described in Belgian Patent No. 848,346, issued May 16, 1977. The N-imidoyl derivatives are covered in Belgian Patent No. 848,545, issued May 20, 1977. The unsaturated side chain-containing compound, also called N-acetyl-dehydrothienamycin or $924A_5$ is a fermentation product of S. cattleya, NRRL 8057, described in Belgian Patent No. 866,035, issued October 17, 1978. Epimeric forms of N-acetyl thienamycin, also called 890A, and $890A_3$, as well as the desacetyl 890A, and desacetyl $890A_3$ are disclosed, respectively in published French Appln. 763,887, filed November 19, 1976, and Belgian Patent 848,349, issued May 16, 1977. Epimeric forms of the unsaturated thienamycin, also called $890A_2$ and $890A_5$,are described in published French of April 28, 1976. The 6-sulfonyloxy-containing N-acetyl compounds, also called $890A_9$ or $890A_{10}$, are disclosed respectively, in published French Appln. 7,734,456, filed November 16, 1977, and published French Appln. No. 7,734,457, filed November 16, 1977. Desacetyl analogues of $890A_9$ and $890A_{10}$ are respectively disclosed in DE-OS 28 05 701, French Appln. 7,803,666, filed February 9, 1978, DE-OS 28 05 724, and also in French Appln. 7,803,667, filed February 9, 1978. Some of these latter compounds in the $890A_9$ and $890A_{10}$ series are also known as derivatives of olivanic acid (see Corbett et al.,

J. Chem. Soc. Chem. Commun. 1977, No. 24, pp. 953-54). Compounds of the Formula II above when $R^4$ is hydrogen, also called descysteaminyl thienamycins, are disclosed in Belgian Patent 867,227, issued November 20, 1978.

When $R^6$ is hydrogen, and X is $CH_2$, these compounds are disclosed in German Off. 2,751,624.1, filed November 18, 1977.

A thienamycin-type antibiotic in which $R^4$ is $-SCH_2CH_2NHAc$ and $R^6$ is $C_2H_5$, has been named PS-5 and is reported by K. Okaimura et al., J. Antibiotics 31 p. 480 (1978), see also Belgian Patent 865,578.

The compounds in which X is S, also called "penems", are described by R.B. Woodward in "Recent Advances in the Chemistry of β-Lactam Antibiotics", J. Elks (Ed), The Chemical Society, London, 1977, p. 167; R.B. Woodward, Abstracts of Uppsala University 500 Years Symposium on Current Topics in Drug Research, Uppsala, Sweden, October 1921, 1977. Acta. Pharm. Suecica, Vol. 14, Supplement, p. 23, and U.S. Patent 4,070,477, issued January 24, 1978.

Particularly preferred members within the thienamycin class of compounds are the N-formimidoyl and N-acetamidoyl derivatives of thienamycin. The crystalline form of N-formimidoyl thienamycin, which has recently been described, is also useful in the practice of this invention. An example illustrating a preferred way of making this compound follows:

ILLUSTRATIVE EXAMPLE

N-Formimidoyl thienamycin, (NFT) crystalline

Step A. Benzylformimidate hydrochloride

A 3 l. three-necked flask fitted with an addition funnel, overhead stirrer, and a reflux condenser, was charged with a mixture of benzyl alcohol (125 g., 1.15 mol) formamide (51 g., 1.12 mol) and anhydrous ether (1200 ml.). The mixture was stirred vigorously at room temperature (20-25°C) under a nitrogen atmosphere and benzoyl chloride (157 g., 1.12 mol) in 50 ml. of anhydrous ether was added dropwise using the addition funnel. The addition required approximately 50 minutes.

The reaction mixture was stirred an additional 60 minutes at room temperature. The ether was removed by decantation and 300 ml. of acetic anhydride in 500 ml. of anhydrous ether was added. The mixture was stirred 30 minutes at room temperature. The precipitate was allowed to settle and the ether-acetic anhydride was again removed by decantation. The solid was collected by filtration, washed with 500 ml. of ether and dried in vacuo over KOH at 25°C for 2 hrs. to give 130 g. (67%) of benzylformimidate hydrochloride as a white solid.

The product was assayed by NMR $\delta$ (DMSO) 5.7 (s, 2H, $\emptyset CH_2$), 7.5 (s, 5H, $\emptyset$), 9.0 (s, 1H, HC=N). The product is thermally unstable. It decomposes to formamide and benzyl chloride at 0°C and above. However, no appreciable decomposition was detected on storage at -20°C for 2 months.

Step B. Derivatization of Thienamycin

Thienamycin (in the form of a 6 l. aqueous solution, pH = 6.5, concentrate from the fermentation broth, containing 28 g. thienamycin) was placed in a large beaker (12 l) and cooled to 0°C. The beaker was equipped with a pH meter and an efficient high speed stirrer. The pH was raised to 8.5 by the careful addition of 3N KOH (KOH was added dropwise via syringe to the stirred solution). The solution was treated with 6 equivalents of solid benzyl formimidate hydrochloride (~100 g.) in portions while maintaining the pH at 8.5 ± 0.3 by the addition of 3N KOH (200 ml.) using a syringe. The addition required 3-5 min. The reaction mixture was stirred for 6 min. at 0°C and then assayed by liquid chromatography to insure completion of the reaction. The solution was adjusted to pH 7 with 1N HCl. The volume of the reaction mixture was measured, and the solution was assayed by UV. The neutralized reaction mixture was concentrated to 15 g./l. on the reverse osmosis unit at <10°C. The volume of the concentrate was measured and the pH was adjusted to 7.2-7.4, if necessary. The concentrate was filtered through a medium porosity sintered glass funnel to remove any solids present after concentration.

Step C. Dowex 50W x 2 Chromatography

The concentrate (750-1000 ml., 15-20 g.) was applied to 0°C. to a precooled 18 l. column of Dowex 50W x 2 in the potassium cycle (200-400 mesh resin) and the column was eluted at 0-5°C with distilled deionized water a flow rate of 90 ml/min. and a head pressure of 0-45 psig.

Forerun fractions of 4 l., 2 l., and one l., were collected followed by 18 fractions of 450 ml. each, and one final fraction of 2 l. Each fraction was assayed by UV (1/100 dilution, $NH_2OH$ extinction was omitted) and the total amount of NFT present in each fraction was calculated. The beginning and end fractions were assayed for liquid chromatography purity and the desired rich cut fractions were combined. The pH of the combined rich cuts was determined by both pH meter and bromothymol blue indicating solutions and was adjusted to pH 7.2-7.4 if necessary. The combined rich cuts (3-4 l.) were then assayed by UV and the total formamidine content was determined, 15-16 g., 75% yield from the column. The rich cuts were concentrated on the reverse osmosis unit at < 10°C as far as possible, then the concentration to 33 g./l. was completed on the circulatory evaporator at less than 28°C. A total volume of about 500 ml. concentrate was obtained.

Step D. Crystallization of N-Formimidoyl Thienamycin

The concentrate from the previous step is adjusted to 7.3, if necessary, and N-formimidoyl thienamycin content assayed by UV, was about 85-90%. The concentrate was filtered through a sintered glass funnel (medium porosity) into a large Erlenmeyer flask. Five volumes (~ 2200 ml.) of 3A ethanol was filtered into the concentrate and the solution was stirred at room temperature for 10 minutes and at 0°C for 12-24 hrs.

The crystals were filtered by suction filtration and washed with 0.1 volume (~250 ml.) of 0°C 80% 3A ethanol followed by 1/25 volume (100 ml.) of 3A ethanol at room temperature. The crystals were dried *in vacuo* for 12-24 hrs. to give approximately a 40% overall yield of N-formimidoyl thienamycin (10-12 g.).

Analytical results on a 50 g. blend of N-formimidoyl thienamycin, prepared as above, are as follows:

| | | | | |
|---|---|---|---|---|
| C, | theory | 45.42%; | found, | 45.82% |
| H, | theory | 6.03%; | found, | 5.72% |
| N, | theory | 13.24%; | found, | 13.10% |
| S, | theory | 10.10%; | found, | 10.14% |

residue on ignition, predicted 0.5, found 0.47%; $[\alpha]_D^{25}$ = 89.4°, T.G. = 6.8%, UV $\lambda$ max 300 MM, E% = 328.

METHODS OF USING THE INVENTION

As mentioned above, the thienamycin-type compound is used in combination with the dipeptidase inhibitor.

The combination of the novel chemical inhibitor of this invention and the thienamycin class compound can be in the form of a pharmaceutical composition containing the two compounds in a pharmaceutically acceptable carrier. The two can be employed in amounts so that the weight ratio of the thienamycin class compound to inhibitor is 1:3 to 30:1, and preferably 1:1 to 5:1.

The components can also be separately administered. For instance, the thienamycin class compound can be administered intramuscularly or intravenously in amounts of 1-100 mg/kg/day, preferably 1-20 mg/kg/day, or 1-5 mg/kg/day, in divided dosage forms, e.g., three or four times a day. The inhibitor can be separately administered, orally, intramuscularly, or IV, in amounts of 1-100 mg/kg/day, or preferably 1-30 mg/kg/day, or 1-5 mg/kg/day. The amounts of the two components administered during one day ideally are within the ratio limits denoted above.

The most preferred dosage regimen and level is the combination of crystalline N-formimidoyl thienamycin and the other being the crystalline form of 7-(L-amino-2-carboxyethylthio)-2-(2,2-dimethylcyclopropanecarboxamido)-2-heptenoic acid, co-administered in a sterile aqueous IV injection form (sodium salt), at a level of 250 or 500 mg of the thienamycin and about 1:1 (weight) of the heptenoic acid, or 250 or 500 mg. This dose is given to humans (each assumed to weigh about 80 kg.) from 1 to 4 times daily, or 3.1-25 mg/kg/day of each drug.

The components, whether administered separately or together are employed in pharmaceutically acceptable carriers such as conventional vehicles adapted for oral adminstration such as capsules, tablets, or liquid solutions or suspensions. The components separately or together, can also be dissolved in a vehicle adapted for administration by injection. Suitable formulations for oral use, may include diluents, granulating agents, preservatives, binders, flavoring agents, and coating agents. The example of an oral use composition in the combination of active ingredients, or the acid component alone, intermixed in the dry pulverulent state with gelatin, starch, magnesium stearate, and alginic acid, and pressed into a tablet.

As noted above, the presently known preferred method is parenteral administration of the thienamycin class compound and either co-parenteral administration or oral administration of the inhibitor compound.

METHODS OF TESTING THE COMBINATION ANTIBACTERIAL AGENT

As noted, disposition studies with thienamycin, its natural analogs and its semi-synthetic derivatives have revealed a major metabolic degradation pathway of elimination in the various species examined (mouse, rat, dog, chimpanzee, Rhesus monkey). The extent of metabolism is reflected in low urinary recovery and short plasma half-lives. The nature of this degradation was demonstrated to be lactam cleavage by the renal dipeptidase (E.C.3.4.13.11), described first by Bergmann, M. and Schleich, H., Z. Physiol. Chem., 205 65 (1932); see also Greenstein, J.P., Advances in Enzymology, Vol. VIII, Wiley-Interscience, (1948), New York, and Campbell, B.J.; Lin, Y-C., Davis, R.V. and Ballew, E., "The Purification and Properties of Particulate Renal Dipeptidase", Biochim. Biophys. Acta., 118, 371 (1966).

In order to demonstrate the ability of the compounds of Formula I to suppress the action of the renal dipeptidase enzyme, an in vitro screen procedure was followed. This measured the ability of compounds to inhibit hydrolysis of glycyldehydrophenylalanine (GDP) by a solubilized preparation of dipeptidase isolated from hog kidneys. The procedure is as follows: to a 1 ml. system containing 50 mM "MOPS" (3-(N-morpholino)propanesulfonic acid) buffer, pH 7.1, is added 5μg of lyophilized enzyme, and the test compound at a final concentration of 0.1 mM. After a five minute incubation at 37°C, GDP is added to a final concentration of 0.05mM. Incubation is continued for 10 minutes, at 37°C and hydrolysis of GDP is measured by the change in optical density with time at 275 nm. Inhibition of the enzyme is gauged by comparison to a standard run containing no inhibitor and is expressed as the inhibitor binding constant, $K_i$. This is the concentration of the inhibitor which achieves 50% inhibition of enzyme.

The substrate GDP is employed in preference to thienamycin in this screen because it has a much higher maximal velocity of hydrolysis by renal dipeptidase, thereby reducing the amount of enzyme required. Both GDP and thienamycin have a similar affinity for renal dipeptidase; furthermore, $K_i$'s of inhibitors tested have been identical for the two substrates.

In addition to this *in vitro* screen procedure, an *in vivo* screen was followed to measure the test compound's ability to inhibit metabolism as reflected by increase in urinary recovery of thienamycin from the mouse. The procedure involves co-administration of the test compound by the intravenous or subcutaneous route at a dose-rate of 10-100 mg/kg, with 10 mg/kg thienamycin. Thienamycin recovery in the urine over a 4 hour period is then compared with its recovery in a control group to which test compound was not co-administered.

Urinary recovery of thienamycin was measured in all cases with the use of a cylinder or disc diffusion assay, conducted in a manner described in U.S. Patent 3,950,357. This bioassay, with *Staphylococcus aureus* ATCC 6538 as the test organism, has a useful response range from 0.04 µg/ml to 3.0 µg/ml.

The inhibitor compounds can be made by condensing directly an ω-bromo or -chloro alkenoic acid compound and cysteine . HCl or 1-amino ethane phosphoric acid.

The bromo intermediate compound has the structure

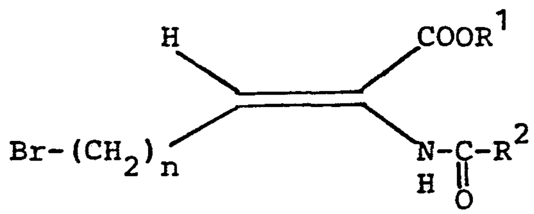

III

wherein n is the number of carbons in the desired alkylene chain (i.e., from 3-7).

Another route for preparing the compounds according to the invention utilizes a chloro-keto ester intermediate

$$Cl-(CH_2)_n-CH_2-\overset{O}{\overset{\|}{C}}-CO_2R^1 \qquad IV$$

in reaction with the desired amide,

$$R^2\overset{O}{\overset{\|}{C}}NH_2 \qquad V$$

in toluene at reflux in the presence of a catalytic amound of p-toluene sulfonic acid. The resulting intermediate is hydrolyzed to the acid; the chloro group is then displaced in reaction with cysteine or 1-amino ethane phosphoric acid. This reaction is valuable since it permits use of the chiral amide V, thereby preparing a functionalized side chain. In addition, the mixture of Z + E isomers prepared after the mercaptan or 1-amino ethane phosphoric acid condensation can be directly resolved into the Z form,

e.g. by adding acid to a pH about 3, and heating to about 90°C for 30 minutes. Only the Z form remains, and recovery is simple and straight forward.

More detail about preparation of the compounds is found in the following examples.

EXAMPLE 1

Z-8-Bromo-2-(2,2-Dimethylcyclopropanecarboxamido)-2-octenoic acid

To a suspension of 14.4 g (0.3 mole) of 50% NaH dispersion in 360 ml of toluene cooled in an ice bath and in a $N_2$ atmosphere was added over 45 min. a solution of 146 g (0.6 moles) of 1,6-dibromohexane and 57.6 g (0.3 mole) of ethyl 1,3-dithiane-2-carboxylate in 120 ml of DMF. The cooling bath was removed and the mixture stirred at room temperature for 20 hrs. The reaction mixture was washed with water (3 x 210 ml), dried over $MgSO_4$ and evaporated under reduced pressure to give 179.5 g of a yellow oil containing the desired alkylated dithiane, 1,6-dibromohexane and mineral oil. This crude material was used in the next reaction without purification.

To a suspension of 426 g (2.4 moles) of N-bromosuccinamide in 800 ml of acetonitrile and 200 ml of $H_2O$ was added over 45 min. a solution of the crude dithiane in 100 ml of acetonitrile. The temperature of the reaction mixture was maintained below 25°C with an ice bath. After stirring at 20°C for 10 min. the dark red reaction mixture was poured into 2 l. of hexane-$CH_2Cl_2$ (1:1). The solution was shaken with saturated $NaHSO_3$ (2 x 400 ml) and water (1 x 500 ml). Then 400 ml of saturated $Na_2CO_3$ solution was added in small portions (vigorous $CO_2$ solution). After the foaming subsided the funnel was shaken and the aqueous phase separated. The organic layer was extracted with saturated $Na_2CO_3$ solution (400 ml) and water (500 ml) and dried over $MgSO_4$. Removal of the solvent under reduced pressure gave 133.8 g of crude bromo ketoester containing 1,6-dibromohexane and mineral oil. This crude material was used in the next reaction without purification.

A mixture of 133.8 g of crude bromo ketoester, 133 ml of 50% hydrobromic acid and 267 ml of acetic acid was heated at 90°C (internal temperature) for 75 min. The dark solution was evaporated under reduced pressure until most of the acetic acid was removed. The residue was dissolved in 500 ml of ether, washed with water (2 x 100 ml) and extracted with saturated $NaHCO_3$ (3 x 200 ml). The combined $NaHCO_3$ extracts were extracted with ether (2 x 100 ml) and acidified with concentrated HCl. The precipitated oil was extracted with ether (3 x 200 ml). The ether extracts were washed with water (1 x 100 ml) and saturated brine (1 x 100 ml) and dried over $MgSO_4$. Removal of the ether under reduced pressure gave 46.2 g of pure bromoketo acid. Homogeneous by TlC (silica gel, 4:1 toluene-acetic acid). The NMR spectrum was consistent with the desired product.

A mixture of 46.1 g (0.194 moles) of the bromoketo acid , 17.6 g (0.156 mole) of 2,2-dimethylcyclopropanecarboxamide and 450 ml of toluene was heated under reflux for 13 hrs., with collection of water in a small Dean-Stark trap. After cooling, the clear reaction mixture was extracted with saturated $NaHCO_3$ solution (4 x 100 ml). The combined extracts were washed with ether (2 x 100 ml) and then the pH was adjusted to 3.5 (pH meter) by addition of concentrated HCl. An oil precipitated which soon crystallized. The solid was filtered, washed well with water and dried. Recrystallization from acetonitrile gave 22.5 g of Z-8-bromo-2-(2,2-dimethylcyclopropanecarboxamido)-2-octenoic acid, m.p. 151-153°C. Homogeneous by TLC (4:1 toluene-acetic acid). The NMR spectrum was consistent with the desired structure.

| Anal. ($C_{14}H_{22}BrNO_3$) | Calcd | Found |
|---|---|---|
| C | 50.61 | 50.66 |
| H | 6.67 | 6.96 |
| N | 4.22 | 4.45 |
| Br | 24.05 | 23.95 |

The following ω-bromo compounds were prepared using the same procedure:

Z-6-Bromo-2-(2,2-dimethylcyclopropanecarboxamido)-2-hexenoic acid;

Z-7-Bromo-2-(2,2-dimethylcyclopropanecarboxamido)-2-heptenoic acid;

Z-9-Bromo-2-(2,2-dimethylcyclopropanecarboxamido)-2-2-nonenoic acid;

Z-10-Bromo-2-(2,2-dimethylcyclopropanecarboxamido)-2-decenoic acid;

Z-8-Bromo-2-(2,2-dichlorocyclopropanecarboxamido)-2-octenoic acid.

## EXAMPLE 2

### Z-7-(L-amino-2-carboxyethylthio)-2-(2,2-dimethylcyclopropanecarboxamido)-2-heptenoic acid

Z-7-bromo-2-(2,2-dimethylcyclopropanecarboxamido)-2-heptenoic acid (prepared as in Example 1) (185 mg, 1.05 mmoles) is dissolved in 2.02 ml NaOH solution (2.0 N), and deoxygenated by bubbling a stream of nitrogen gas through it for a minute. Then cysteine.HCl (185 mg, 1.05 mmoles) is added all at once and the reaction stirred at room temperature in a $N_2$ atmosphere for 3 hours. The reaction mixture is applied to 2 x 20 cm column of Dowex 50 x 4 (100-200 mesh $H^+$), and eluted with 300 ml $H_2$, then 200 ml of 2N $NH_3$ solution. Ammonia is evaporated under reduced pressure to give 284 mg of a yellowish glass. This product is dissolved in 4 ml ethanol, and the insoluble material filtered.

The filtrate is added dropwise to rapidly stirred diethylether (150 ml). The solid which precipitates is filtered, washed with ether and dried to yield 171 mg product, having one spot (ninhydrin positive) in TLC (nBuOH, HOAc, $H_2O$; 4:1:1) rf. about 6; NMR is consistent in the desired structure.

| Anal. | ($C_{16}H_{26}N_2O_5S$) | Calcd. | Found |
|---|---|---|---|
| | C | 53.61 | 52.55 |
| | H | 7.31 | 7.40 |
| | N | 7.81 | 7.89 |
| | S | 8.94 | 9.63 |

EXAMPLE 3

Sodium Z-7-(L-amino-2-Carboxethylthio)-2-(2,2-dimethylcyclopropane carboxamido)-2-heptenoic acid

A. Grignard Preparation of Ethyl-7-chloro-2-oxoheptanoate

Equimolar amounts (8 moles each) of 1-bromo-5-chloropentane and magnesium are reacted in tetrahydrofuran (960 ml) at 25°C. The flask is charged with Mg in the THF and the bromochloropentane added over 1 hr, then aged 2 hrs. After the reaction was judged complete, the reaction solution was added (cooled to -15°C) to 16 moles of diethyloxalate in 1856 ml tetrahydrofuran, while maintaining the temperature at -10°C. 3 N HCl was added to quench, keeping the temperature below 25°C. After stripping solvents, the calculated yield is 48.8% of the ethyl-1-chloro-6-oxoheptenoate.

B. Condensation and Hydrolysis

S-2,2-dimethylcyclopropyl carboxamide (1017 g), 2143.6 g of ethyl-7-chloro-2-oxoheptanoate, 9 liters of toluene and 12 g of p-toluene sulfonic acid were charged to a 22 L flask, and heated to reflux with stirring. After 23 hrs., liquid chromatography showed the expected product ratio, and 4 L of toluene were

removed under slightly reduced pressure. The pot was charged with water, neutralized to pH 7 with 2N NaOH, and vacuum distilled leaving a final pot volume of about 5 liters.

This was hydrolyzed by adding 1760 g of 50% aq. NaOH (4 liters water) and stirring overnight. The flask was charged with 4 L methylene chloride, and pH adjusted to 8.8 using HCl. unreacted amide crystallized out. The organic layers were separated from water, and then evaporated. The gummy residue was dissolved in 8 liters water containing 720 g 50% NaOH, and to this solution was charged 1818 g L. cysteine-HCl.$H_2O$, 2 kg ice, 2484 g 50% NaOH and 1 liter water.

The pH of this solution, after aging overnight at room temperature, is adjusted to 3.0 with conc. HCl, and the resulting gummy suspension heated to 95°C to afford a clear solution. After 30 minutes, no E isomer could be detected by liquid chromatography. After work-up and purification, the overall yield was 50%. This material was recrystalized from acetonitrile 1500 g of the recrystalized material was dissolved in 6 liters water and 910 ml 3.88 N NaOH, then neutralized to pH 7, and lyophilized to afford 1569 g (98.6%) of the title compound; Analysis:
calcd: C, 50.52; H, 6.62; N, 7.36; S, 8.43; Na, 6.04; found: C, 50.71; H, 6.78; N, 7.49; S,8.52; Na, 5.92.

EXAMPLE 4

Z-2-(2,2-dimethylcyclopropane carboxamido-8-/1-(phosphono)ethylamino/-2-octenoic acid

335.1 mg Z-8-bromo-2-(2,2-dimethylcyclopropane carboxamido)-2-octenoic acid is reacted with 138.2 mg 1-amino ethane phosphoric acid and 435 mg $Na_2CO_3$ in 5 ml water, heated to 60°C for 10 hours under nitrogen, cooled, then purified through a Dowex 50 X 8 $H^+$ column, eluting with water. NMR confirmed presence of product, Ki = 0.14.

WHAT WE CLAIMED IS:

1. Compounds of the general formula

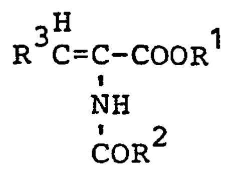

I

in the Z configuration wherein $R^2$ is 2,2-dimethylcyclopropyl; $R^1$ is hydrogen, loweralkyl of 1-6 carbon atoms, dialkylaminoalkyl with 1-6 carbon atoms in each alkyl group, or a pharmaceutically acceptable cation; $R^3$ is an alkylene chain of 3-7 carbon atoms, having a terminal substituent which is 2-amino-2-carboxyethylthio or 1-(phosphono)ethylamino.

2. A compound of Claim 1 which is Z-8-(L-2-amino-2-carboxyethylthio)-2-(2,2-dimethylcyclopropanecarboxamido)-2-octenoic acid.

3. A compound of Claim 1 which is Z-7-(L-2-amino-2-carboxyethylthio)-2-(2,2-dimethylcyclopropanecarboxamido)-2-heptenoic acid.

4. A compound of Claim 1 which is Z-2-(2,2-dimethylcyclopropanecarboxamido)-8-[1-(phosphono)ethylamino]-2-octenoic acid.

5. The compound of Claims 2, 3 or 4 in the sodium, potassium, calcium or magnesium salt form.

6. An antibacterial composition comprising a thienamycin type compound and a compound according to anyone of Claims 1 to 5.

7. The antibacterial composition of Claim 6 comprising Z-7-(L-2-amino-2-carboxyethylthio)-2-(2,2-dimethylcyclopropanecarboxamido)-2-heptenoic acid or its sodium, potassium, magnesium or calcium salt and N-formimidoyl thienamycin.

## Claimsfor Austria

1. Process for preparing compounds of the general formula

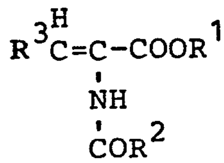

I

in the Z configuration, wherein $R^2$ is 2,2-dimethylcyclopropyl; $R^1$ is hydrogen, loweralkyl of 1-6 carbon atoms, dialkylaminoalkyl with 1-6 carbon atoms in each alkyl group, or a pharmaceutically acceptable cation; $R^3$ is an alkylene chain of 3-7 carbon atoms, having a terminal substituent which is 2-amino-2-carboxyethylthio or 1-(phosphono)ethylamino, which comprises either

a) condensing directly an ω-bromo or ω-chloro alkenoic acid compound of the general formula

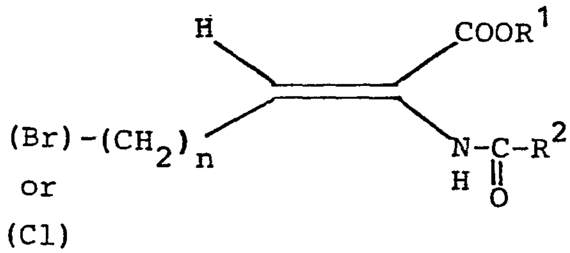

wherein n is from 3-7
and cysteine . HCl or 1-amino ethane phosphoric acid or

b) reacting a chloro-keto ester intermediate

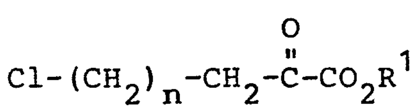

with the desired amide

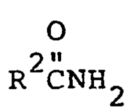

,

hydrolyzing the resulting intermediate to the acid and displacing the chloro group by reaction with cysteine or 1-amino ethane phosphoric acid.

2. Process according to Claim 1 for preparing Z-8-(L-2-amino-2-carboxyethylthio)-2-(2,2-dimethylcyclopropanecarboxamido)-2-octenoic acid.

3. Process according to Claim 1 for preparing Z-7-(L-2-amino-2-carboxyethylthio)-2-(2,2-dimethylcyclopropanecarboxamido)-2-heptenoic acid.

4. Process according to Claim 1 for preparing Z-2-(2,2-dimethylcyclopropanecarboxamido)-8-[1-(phosphono)ethylamino]-2-octenoic acid.

5. Process according to Claim 1 for preparing the compounds of Claims 2, 3 or 4 in the sodium, potassium, calcium or magnesium salt form.

0048301

European Patent Office

# EUROPEAN SEARCH REPORT

Application number

EP 80 10 5739.9

## DOCUMENTS CONSIDERED TO BE RELEVANT

| Category | Citation of document with indication, where appropriate, of relevant passages | Relevant to claim |
|---|---|---|
| X | EP - A1 - 0 007 614 (MERCK) | |
| | * claims 1 to 5 * | 6-7 |
| | * page 62, line 12; example 18 * | 1-5 |
| | * page 63, line 17; example 19 * | 1-5 |
| | ---- | |

**CLASSIFICATION OF THE APPLICATION (Int. Cl.³)**

C 07 C 149/243
C 07 F 9/38
A 61 K 31/195
A 61 K 31/66

**TECHNICAL FIELDS SEARCHED (Int. Cl.³)**

A 61 K 31/195
A 61 K 31/66
C 07 C 149/243
C 07 F 9/38

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 03-06-1981 | BREW |

EPO Form 1503.1 06.78